# EUROPEAN PATENT SPECIFICATION

(11) **EP 0 882 134 B1**
(45) Date of publication and mention of the grant of the patent: **22.04.2009**
(21) Application number: 97906764.2
(22) Date of filing: 20.02.1997
(51) Int. Cl.: C12N 15/86, A61K 48/00, C12N 7/04, C12N 15/19, C07K 14/16, C12N 5/10, C12N 5/06, A61K 51/12

(54) **METHODS AND COMPOSITIONS FOR PROTECTIVE AND THERAPEUTIC GENETIC IMMUNIZATION**
VERFAHREN UND ZUSAMMENSETZUNGEN ZUR GENETISCH SCHÜTZENDEN UND THERAPEUTISCHEN IMMUNISIERUNG
PROCEDES ET COMPOSITIONS POUR L'IMMUNISATION GENETIQUE PROTECTRICE ET THERAPEUTIQUE

(30) Priority: 21.02.1996 US 604627
(43) Date of publication of application: 09.12.1998
(73) Proprietor: Genetic Immunity kft., Budapest 1045 (HU)
(72) Inventor: Genetic Immunity kft., Budapest 1045 (HU)
(74) Representative: Jones, Helen M.M.
(86) International application number: PCT/US1997/002933
(87) International publication number: WO 1997/031119

(56) References cited:
- WO-A-92/20356
- WO-A-96/06177
- WO-A1-94/24267
- GENE THERAPY OF CANCER : 2ND EUROPEAN CONFERENCE, LONDON, UK, SEPTEMBER 7-8 1995 HEEMSKERK, MHM et al.: "Melanoma-specific CTL, generated in viro using dendritic cells expressing melanoma associated antigens". Page A13 XP000675471
- JOURNAL OF IMMUNOLOGY, vol. 154, May 1995, BALTIMORE US, pages 4685-4692, XP000676343 WANG, M. ET AL.: "Active immunotherapy of cancer with a nonreplicating recombinant Fowlpox Virus encoding a model tumor-associated antigen"
- TRANSPLANTATION, vol. 59, no. 4, 27 February 1995, pages 544-551, XP000675701 THOMSON, A.W. ET AL.: "In vitro propagation and homing of liver-derived dendritic cell progenitors to lymphoid tissues of allogeneic recipients"
- AIDS RESEARCH AND HUMAN RETROVIRUSES, vol. 9, no. SUPPL. 01, 1 October 1993, page S33 XP000560222 KLEIN M ET AL: "DEVELOPMENT OF A CROSS-NEUTRALIZING HIV-1 PSEUDOVIRION-BASED VACCINE"
- JOURNAL OF VIROLOGY, vol. 69, no. 1, 1 January 1995, pages 376-386, XP000560257 WISKERCHEN M ET AL: "HUMAN IMMUNODEFICIENCY VIRUS TYPE 1 INTEGRASE: EFFECTS OF MUTATIONS ON VIRAL ABILITY TO INTEGRATE, DIRECT VIRAL GENE EXPRESSION FROM UNINTEGRATED VIRAL DNA TEMPLATES, AND SUSTAIN VIRAL PROPAGATION IN PRIMARY CELLS"
- LISZIEWICZ J. ET AL.: 'induction of potent human immunodeficiency virus type 1 specific T-cell restricted immunity by genetically-modified dendritic cells' J. VIROL. vol. 75, no. 16, 2001, pages 7621 - 7628
- LISZIEWICZ J. ET AL.: 'DermaVir: a novel topical vaccine for HIV/AIDS' J. INVEST DERMATOL. 2004, pages 1 - 10
- LISZIEWICZ J. ET AL.: "Control of viral rebound through therapeutic immunisation with DermaVir" AIDS, vol. 19, 2005, pages 35-43,
- J. LISZIEWICZ ET AL.: 'Control of HIV despite the discontinuation of antiretroviral therapy' NEW ENGLAND J. MED vol. 340, no. 21, 1999, pages 1683 - 1684

### SEQUENCE LISTING

(1) GENERAL INFORMATION:
   (i) APPLICANT: Lisziewicz, Julianna Lori, Franco
   (ii) TITLE OF INVENTION: METHODS AND COMPOSITIONS FOR PROTECTIVE AND THERAPEUTIC GENETIC IMMUNIZATION
   (iii) NUMBER OF SEQUENCES: 2
   (iv) CORRESPONDENCE ADDRESS:
      (A) ADDRESSEE: Saliwanchik & Saliwanchik
      (B) STREET: 2421 N.W. 41st Street, Suite A-1
      (C) CITY: Gainesville
      (D) STATE: Florida
      (E) COUNTRY: USA
      (F) ZIP: 32606
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: Saliwanchik, David R.
      (B) REGISTRATION NUMBER: 31,794
      (C) REFERENCE/DOCKET NUMBER: RGT-100
   (ix) TELECOMMUNICATION INFORMATION:
      (A) TELEPHONE: (352) 375-8100
      (B) TELEFAX: (352) 372-5800
(2) INFORMATION FOR SEQ ID NO:1:
   (i) SEQUENCE CHARACTERISTICS:
      (A) LENGTH: 27 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:1:
      CTACTATGGT ACCCCAAAGG TGTGCTC 27
(2) INFORMATION FOR SEQ ID NO:2:
   (i) SEQUENCE CHARACTERISTICS:
      (A) LENGTH: 21 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:2:
      TGAATTTTAA AAGAAGGGGA G 21

## Description

### Background of the Invention

Many of the most deadly and debilitating diseases afflicting humans and animals are caused by viruses. The invention described herein is directed toward preventing and treating various diseases caused by viruses, and in particular diseases caused by retroviruses. For example, the subject invention is applicable to cancers such as adult T-cell leukemia/lymphoma, caused by the retrovirus HTLV-1 (human T-cell lymphotropic virus, type 1). This cancer poses a major health problem in Japan and similar problems in clusters of the Caribbean, South American, and United States population. Pombo de Olivera et al. (1990) Lancet 336:987-991; Shermata et al. (1992) Arch Neurol. 49:1113. HTLV-1 has also been linked to various inflammatory disorders, such as Sjögren's syndrome, in western Japan where HTLV-1 infection is endemic (Kaoru et al. [1994] Lancet 344:1116-1119), and degenerative encephalopathy that paralyzes the lower extremities (Dixon et al. [1990] West J. Med. 152: 261-267).

Another retroviral disease addressed specifically herein is AIDS, caused by HIV (human immunodeficiency virus), a particular type of retrovirus called a lentivirus. Recently, it appears that heterosexual women between the ages of 18-24 are the population exhibiting the greatest increase in sero-positivity for HIV. Because women infected with HIV are the major source of infection for infants, experts report that AIDS mortality in children will increase. Kimball et al. (1995) Annu. Rev. Public Health 16:253-282, review the devastating effect of the AIDS epidemic.

Example of retroviruses causing animal diseases include simian immunodeficiency virus (SIV), feline leukemia virus (FeLV), feline T-lymphotropic lentivirus (now designated as feline immunodeficiency virus (FIV) reported by Pedersen et al. [1987] Science 235:790-793), and Bovine leukemia. Other diseases caused by non-retroviruses, which are nevertheless subject to the invention described herein, include cervical cancer caused by human papillomavirus (HPV), primary hepatocellular carcinoma caused by hepatitis B, Burkitt's lymphoma and nasopharyngeal carcinoma caused by Epstein-Barr virus (EBV), lower respiratory infections caused by respiratory syncytial virus, herpes, smallpox, encephalitis, measles, and influenza.

Briefly, viruses are obligatory intracellular parasites that replicate by hijacking the infected host's cellular mechanisms. Virus particles typically include a genome comprising one or a few molecules of either DNA or RNA, or both, and a small number of proteins which form a capsid or which are present as enzymes or regulatory proteins. Viruses are often categorized according to (1) the kind of nucleic acid that constitutes the genome; (2) the strategy of viral replication; and (3) the morphology of the virion. Viral classification and nomenclature are reviewed in Fenner et al., eds. (1993) Veterinary Virology, 2nd ed, Academic Press, Inc., San Diego.

The body combats viral infection with a combination of cellular and humoral immune mechanisms. Cytotoxic T lymphocytes (CTLs) comprise the principle weapon in defending against established viral infection. Specifically. CD8⁺ CTLs recognize antigens associated with class I Major Histo-compatibility (MHC) molecules while CD4⁺ T-celles recognize antigens associated with the class II MHC molecules. Early in the course of viral infection, specific antibodies prove an important weapon in defense against viral attack. Although the precise mechanisms of how antibodies neutralize viruses is not known, IgG, IgA and IgM antibodies have been described which are capable of neutralizing viral particles. Accordingly, the success of prophylactic vaccination with subunit, attenuated, or killed viruses is largely related to the vaccine ability to stimulate antibody responses. See generally Abbas et al. [1994] Cellular & Molecular Immunology, 2nd ed., W.B. Saunders Co., Philadelphia.

More specifically, subunit vaccines consisting of viral proteins are administered in hopes that an immune response raised to viral proteins will effectively combat viral this mode of vaccination is directed at humoral or systemic immune responses and typically elicits the production of IgG antibodies. This type of antibody remains primarily in the bloodstream of the individual in order to prevent blood-born disease. In the case ofHIV-1 for example, many of the vaccines directed at preventing HIV-1 infections are such subunit vaccines which use either recombinant HIV-1 envelope or core proteins and are administered either intramuscularly or subcutaneously. These vaccine approaches have not raised efficient immune responses in HIV models, however, in part because the selected cloned and expressed proteins cannot present viral epitopes in an authentic fashion. Letvin (1993) New Eng. J. Med. 329(19): 1400-1405. Additionally, viral mutation facilitates viral escape from immune responses focused on a few specific epitopes. Nowak et al. (1995) Nature 375: 606-611.

The article entitled "Melanoma-specific CTL, generated in vitro using dendritic cells expressing melanoma associated antigens" from the 2nd European conference on the Gene Therapy of Cancer, London, UK, September 7-8 1995 by Heemskerk, MHM et al discusses the transduction of dendritic cells *in vitro* with retroviral vectors encoding MAGE-1 and tyrosinase, as well as the marker gene lac Z. The cells are tested for their ability to generate MAA-specific CTL, but no *in vivo* work is described.

Wong et al in Journal of Immunology 154 [1995]: 4685-4692 discusses the alteration of a fowl pox virus, which is known to be unable to cause disease in mammalian cells, to include a tumour-associated AGS. A live virus particle is used as the vector for carrying the disease of interest.

WO94/24267 discloses the use of retroviral particles for gene delivery. Nucleotides encoding an antigen and a costimulatory factor are incorporated into a retroviral vector for the purpose of raising an immune response.

Wiskerchen M. et al in Journal of Virology 69(1) [1995]:376-386 explore the integrase coding region of HIV-1. Mutations are made throughout the integrase coding region of HIV-1, and the resulting mutant viruses are classified according to their ability to reproduce. The authors conclude by recommending that chemotherapy targeted to retrovirus integrase may have clinical relevance for HIV-1 infection *in vivo,* but do not discuss raising an immune response in a host.

Remaining with the HIV model, in addition to subunit vaccines, live attenuated viruses have been proposed as vaccines. However, live attenuated viruses that are not able to replicate efficiently *in vivo* do not elicit protective immune responses (Letvin et al [1995] J. Virol. 69:4569-4571). Conversely, state-of-the-art live attenuated HIV viruses that are able to replicate efficiently *in vivo,*
are too dangerous to use for vaccine purposes (Letvin [1993] New Eng. J. Med. 329(19):1400-1405).

A particular example of an attenuated live vaccine that proved too dangerous for general vaccine purposes was developed against rhesus macaque SIV (SIVmac). The attenuation involved a deletion of the viral nef gene, a 25-29 kD protein located in the infected cell's cytoplasm and plasma membrane (Allan, J.S., et al. [1985] Science 230:810-813). The nef-deleted SIVmac maintained the ability to integrate and replicate. Although nef-deleted SIVmac vaccination protected adult rhesus monkeys against subsequent infection with pathogenic SIVmac (Daniel et al. [1992] Science 258:1938-1941; Almond et al. [1995] Lancet 345:1342-1344), young rhesus monkeys developed sometimes fatal disease upon vaccination with the nef-deleted SIVmac. Baba et al. (1995) Science 270:1220-1221.

Moreover, it has been shown recently (Heath et al. [1995] Nature 377:740-744) that HIV-1 complexed with neutralizing antibodies is .highly infectious in the presence of follicular dendritic cells. This explains why a traditional vaccine strategy inducing a humoral response may not be effective.

Thus, the possibility of contracting disease is indeed the major concern related to the use of replication-competent attenuated viruses. Additionally, in the case of retroviruses (especially lentiviruses), it can take years before understanding the potential toxicity of traditional vaccines. This greatly increases the safety concerns and limits the use of replication competent virus vaccines. There is an urgent need to identify safe and effective means for combating viral infections. This need is addressed by the genetic immunization technique of the present application, which provides protection from a variety of diseases.

### Brief Summary of the Invention

The subject invention concerns uses of immunogenic genetic constructs, uses of dendritic cells and pharmaceutical compositions for raising protective and therapeutic immunity to lentiviral infections in humans and animals by the technique of genetic immunization. Genetic immunization comprises the expression of genes encoding foreign antigens in the lymphoid organs for the prevention and treatment of diseases. As described more fully below, when the genes of a replication-incompetent lentivirus are expressed in the lymphoid organs, preferably in dendritic cells, immunity is generated by primary and secondary immune responses due to viral particle production and limited reinfection.

The invention, in a first aspect, is use of an immunogenic genetic construct which expresses a replication-incompetent lentivirus in a host's lymphoid organ, for the manufacture of a medicament for raising an immune response in the host to the lentivirus.

The invention, in a second aspect, is use of a dendritic cell transduced with an immunogenic genetic construct which expresses a replication-incompetent lentivirus in a host's lymphoid organ for the manufacture of a medicament for raising an immune response in the host to the lentivirus.

The invention, in a third aspect, is a pharmaceutical composition comprising a dendritic cell which has been transduced with an immunogenic genetic construct which comprises plasmid DNA that expresses a replication-incompetent lentivirus, for use in a method of immunization. Preferred aspects are as set out in the dependent claims.

In a preferred embodiment, the uses of the subject invention involve transducing a cell of a host human or animal with a genetic construct which confers upon the transduced cell the ability to express an attenuated virus. The attenuated virus produced by the transduced cell is, infectious but replication-incompetent so that the virus is not capable of pathogenicity. Although the virus produced by the transduced cell is not pathogenic, the attenuated virus elicits an immune response capable of conferring upon the host protection against infection by the wild-type virus. This unique result is accomplished by having the attenuated viruses selectively produced by cells in the lymphoid organs of the host animal or human. Advantageously, the attenuated virus has the size and other characteristics of the natural virus with the exception that the attenuated virus is not capable of pathogenicity. The efficiency of the immune response is maximized by the natural characteristics of the attenuated virus as well as its expression in the lymphoid organs.

The lack of pathogenicity of the attenuated virus produced according to the subject invention can be accomplished in a variety of ways, but, as described herein, preferred embodiments of the subject invention involve genetic modification of the viral genome, e.g., elimination or reduction of the virus' ability to productively replicate or integrate. By cloning this replication-incompetent viral DNA, it is possible to create a virus particle which is only mildly infectious. If presented to a host using standard procedures, such a mildly infectious virus would not stimulate an effective immune response capable of protecting the host against subsequent challenge. In order to solve this lack of protective or therapeutic immunogenecity, the subject invention comprises a unique means of effectively enhancing the immunogenecity of the attenuated viral particle by presenting these viral particles more directly to the immune system components which are responsible for mounting the antiviral response. Specifically, cells within the lymphoid organs are transduced to confer upon them the ability to produce the replication-incompetent viral particles. These viral particles are nearly identical to wild-type virus in terms of size, biochemical properties, and antigen presentation of viral proteins, with the exception that the attenuated virus is not capable of causing active infection. Although the attenuated virus produced by the virus-producing cells of the subject invention is not able to spread the infection, enough virus is produced to elicit an effective immune response. The ability to raise the desired immune response is due to the virion's structural and biochemical similarity to the wild-type virus, as well as the fact that the attenuated virus is specifically produced in close proximity to the appropriate cells of the immune system.

The uses of the subject invention can elicit a protective immune response prior to viral exposure, or, advantageously, after viral exposure to generate an enhanced immune response. Post-exposure treatment is particularly advantageous in the case of infection by viruses which do not compromise the host's immune response, or in the case of non-immunized patients in immunologically competent stages of the disease.

As described more fully herein, the transduction of the host cells can have other highly advantageous consequences including, for example, the inhibition of replication of wild-type virus in the transduced cells. Cross-protection against related viruses can also be achieved because of the presentation of the broadest spectrum of immunologic determinants. Thus, the uses described herein can raise immune responses against heterologous viruses, or even raise a general antiviral immune response. The procedures used in the subject invention are particularly advantageous because they result in a highly effective antiviral immune response while maximizing the safety of the vaccination or therapeutic procedure. According to the subject invention, it is now possible to safely raise an immune response to a full-sized viral particle without danger of causing disease. Furthermore, because viruses are known'to mediate many types of carcinogenesis, the procedures of the subject invention can be used to treat or prevent these cancerous conditions by eliminating or reducing the viral infection which is necessary for the initiation or progression of the cancerous condition. Moreover, the procedures of the subject invention are applicable to treating tumors exhibiting particular recognizable antigens by eliciting the appropriate immune response against said antigens.

In a further embodiment of the use of the subject invention, the immune response generated in the host animal by the expression of the attenuated virus can be enhanced, directed or modulated by introducing in the transduced cells a second genetic construct which directs the expression of an immune system modulating molecule such as a cytokine.

The uses of the subject invention can also generally increase the efficiency of recombinant viral vaccines. For example, substantial research efforts have been made to develop recombinant vaccines using various pox viruses as viral vectors. These pox viruses may be, for example, vaccinia, fowlpox, canary pox, or swinepox. When used in alternative hosts, or when expressing antigens of low immunogenecity, these viral vaccines are often ineffective in eliciting a useful immune response. This problem can be remedied by the unique vehiculating methods of the subject invention whereby transduced cells migrate to the lymphoid organs to express the viral particles. The transduction of the cells migrating to the lymphoid organs greatly increases the efficiency of the immune response elicited by the recombinant viral constructs. Alternatively, these viruses injected directly in the lymphoid organs can infect antigen-presenting cells and produce infectious virus particles. Such *in vivo* injection may overcome the problem of ineffective CTL induction after intradermal administration.

Thus, the uses and compositions of the subject invention are useful for (a) stimulating an effective immune response to viral antigens; (b) inhibiting virus replication and virus-mediated carcinogenesis; (c) generating long-term cross- reactive memory; (d) active immunization against tumors; and (e) generating safe genetic vaccines.

The subject invention combines both safety and efficacy by using attenuated viruses which cannot spread infection. These viruses are made effective by vehiculating them into humans and animals by means other than their own replication. However, if the attenuated virus is capable to complete several rounds of replication, the virus may be injected directly into the lymphoid organs. Consequently the viruses are safe and effective for raising protective and therapeutic immunity in humans and animals.

### Brief Description of the Drawings

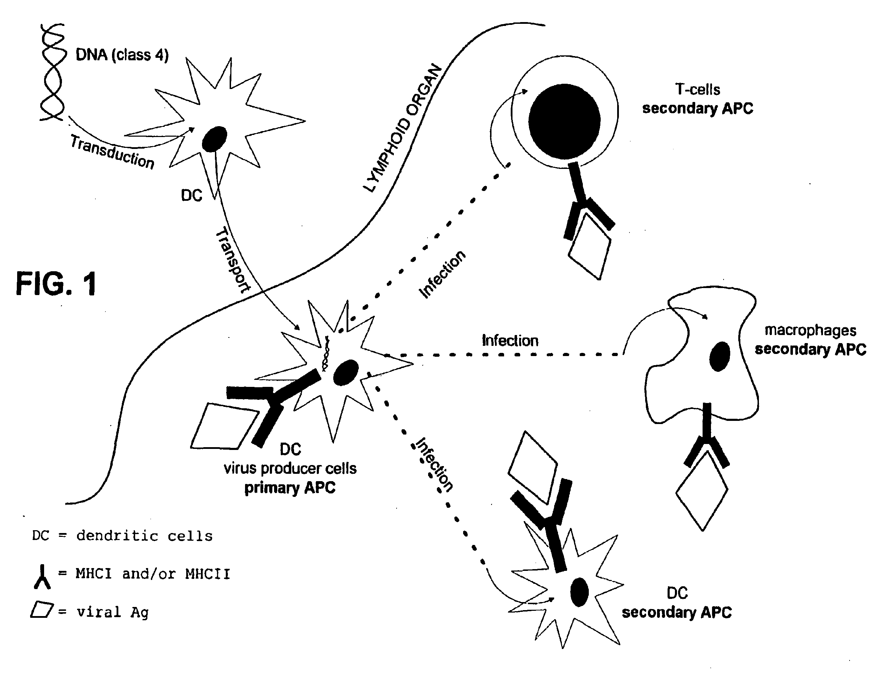

**Figure 1** illustrates genetic immunization. DNA encoding a Class 4 virus is used to transduce cells , preferably dendritic cells (DC). Those transduced cells are then transported to the lymphoid organ, where the transduced cells express the Class 4 DNA as proteins and virus and become primary antigen-presenting cells (APCs). The Class 4 virus, released from the transduced cells, can act as soluble antigen and also infect other cells present in the lymphoid organ such as T-cells, macrophages, and dendritic cells. These infected cells become the secondary antigen-presenting cells. Both primary and secondary APCs present viral antigens in an authentic fashion in the context of the major histocompatibility complex (MHC), concentrated in the lymphoid organ, efficiently raising the host's immune response.

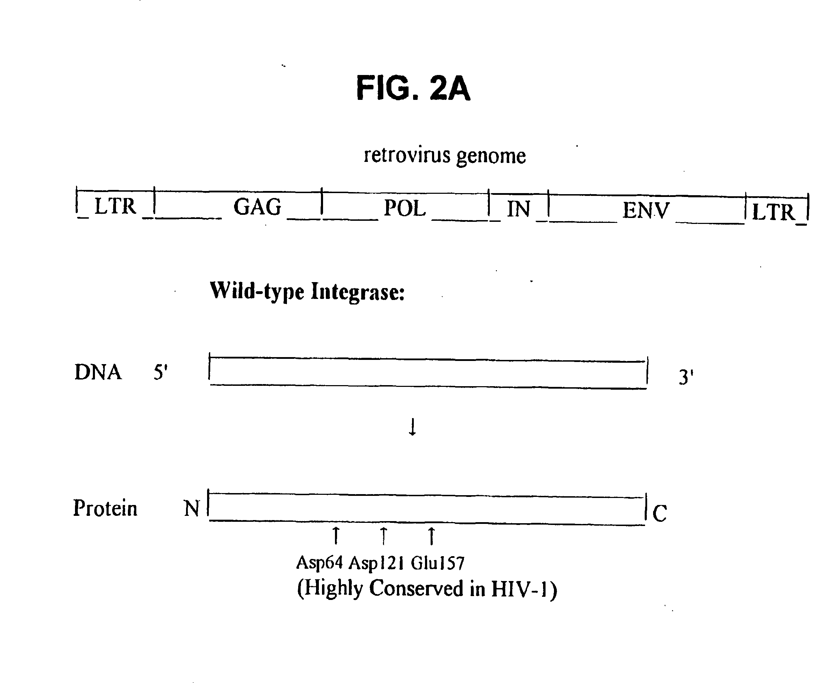

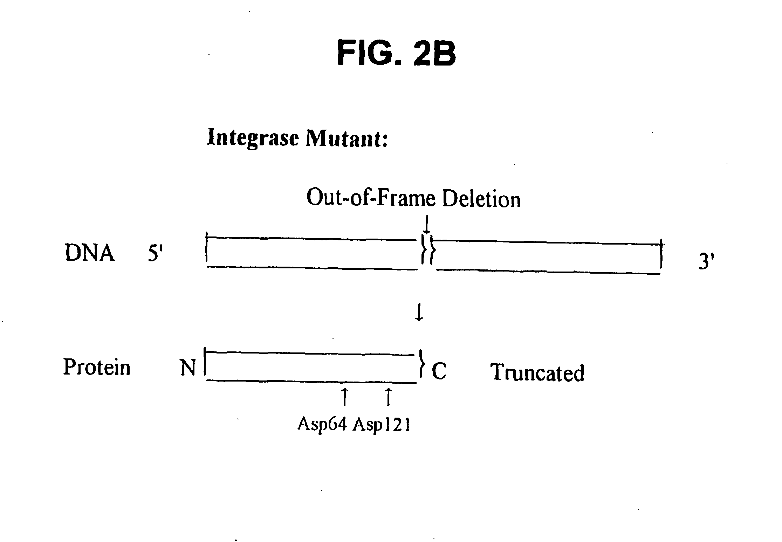

**Figures 2A-2B** illustrate a wild-type retrovirus genome, some particulars of the integrase protein, and an example of the attenuated integrase gene that generates a Class 4 retrovirus. Specifically, this mutation results in a truncated integrase protein which starts at the 5'-end as "wild-type" and stops at the first stop codon after the deletion, consequently the mutant protein is about half size of the wild-type integrase.

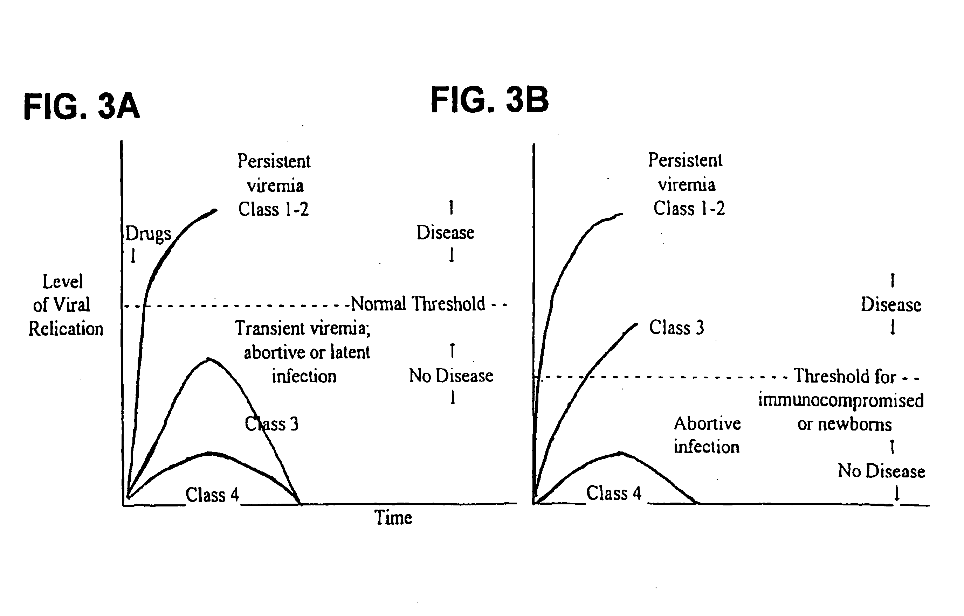

**Figures 3A-3B** depict the pathogenicity of four classes of viruses based on their ability to replicate in a given host Figures 3A-3B are read in conjunction with Table 1. Classes 1-3 have been introduced by Baba et al. (1995) Science 270:1220-1221, and Class 4 is introduced herein. Class 1 virus is wild-type virus that replicates above the threshold level so that persistent viremia exists and causes disease in the host. Class 2 virus may be wild-type or attenuated so that viral replication occurs above the threshold level, but may or may not cause disease in a normal host. Class 3 viruses cause disease in hosts whose immune systems present a lower threshold. Class 4 viruses are attenuated such that replication is self-limiting and the virus can not cause disease even in a host with a lowered threshold. See Example 1, below, for a thorough discussion of Figures 3A-3B.

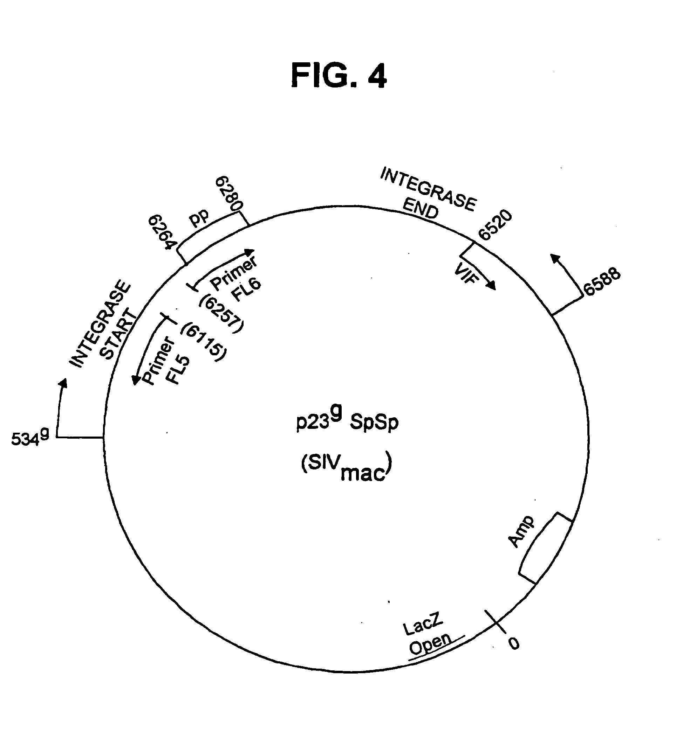

**Figure 4** depicts the plasmid construct p239SpSp, carrying the defective gene encoding the truncated integrase protein.

### Brief Description of the Sequences

SEQ ID NO.1 is primer FL5 as used according to the subject invention.

SEQ ID NO.2 is primer FL6 as used according to the subject invention.

### Detailed Disclosure of the Invention

The present invention concerns uses and compositions for stimulating a safe and effective antiviral immune response in a human or animal. The uses of the subject invention comprise transduction of cells with an immunogenic genetic construct that directs the expression of an attenuated virus. As used herein, reference to "transduction" of cells means the transfer to cells of DNA which confers upon the cells the ability to produce a virus as described herein and, in some instances, express a cytokine and/or tumor antigen. The transduced cells are referred to herein as virus producer cells. Typically, the transduced cells would be cells of an animal or human host. The attenuated virus construct is specifically designed to express an attenuated virus which is not capable of causing disease. The attenuated virus is replication-incompetent. Integration defective viruses are used preferably as replication-incompetent viruses. Although the attenuated virus is not pathogenic, its expression by the virus producer cells is accomplished in a manner which causes a highly efficient and effective immune response in the host. The virus producer cell is designed to produce defective viruses in the lymphoid organs where immune responses against viral antigens are induced. As used herein, references to lymphoid organs would include, for example, lymph nodes, the spleen, and the thymus. A critical aspect of the subject invention is the ability to present to the immune system viral particles which are nearly identical to the natural virus except that the viral particles of the subject invention are not pathogenic. Advantageously, the viral antigens are presented to the immune system in their natural conformation, thereby facilitating a highly effective immune response.

In a preferred embodiment, the viral particles are not pathogenic because they are never present in sufficient numbers to cause disease and/or they cannot establish a self-sustained infection *(i.e.,* Class 4 viruses as described below in Table 1 and its accompanying text). Although the viral particles produced according to the subject invention are not able to cause disease, they are presented in a manner which causes an effective immune response. Specifically, the viral particles are released from the transduced cells in close physical proximity to the immune system cells which are responsible for generating antiviral responses. By creating virus particles which closely mimic the natural virion and by selectively concentrating these non-pathogenic virion particles at the locations where antiviral responses occur, it is possible to create a highly efficient and effective immune response. Advantageously, the virus and the virus producer cells produced according to the subject invention will be ultimately eliminated from the body by the immune system because (a) the virus cannot establish infection and (b) the virus induces an antiviral immune response.

The uses of the subject invention can be prior to the host being infected so as to raise an immune response which will help prevent infection. Alternatively, or additionally, the uses of the subject invention can enhance the host's immune response once a viral infection has occurred. Such post-exposure treatment can be very important, particularly when the viral infection is one which normally would cause a decrease in the host's natural immune response. The enhanced efficiency of the subject invention in generating an antiviral immune response is due in part to a unique method for vehiculating attenuated viral particles within the host human or animal. Specifically, the attenuated non-pathogenic viral particles of the subject invention which produce the therapeutic or preventative immune response are generated by cells in the lymphoid organs such that the release of the viral particles occurs in the location within the human or animal body where antiviral responses are generated by cells of the immune system. The release of the virus particles in the lymphoid organs is achieved by transducing host cells of the lymphoid organs (or cells which migrate preferentially to the lymphoid organs) with a genetic construct which results in expression of the attenuated virus. As described more fully herein, transduction of dendritic cells is particularly preferred.

A further aspect of the subject invention concerns the stimulation or direction of a specific immune response of the host by co-introduction into the virus producer cells of at least one gene expressing an immune modulating molecule in addition to the genetic material encoding the attenuated virus. This creates a virus/cytokine producer cell. The virus/cytokine producer cells are designed to express gene products in the lymphoid organs. The cytokine augments and/or influences the type of immune response generated by the attenuated virus.

Examples of cytokines which can be used to enhance the immune response or direct a particular immune response include, but are not limited to, IL-1, IL-2, IL-3, IL-4, IL- 6, IL-7, IL-12, TNF, GM-CSF, and IFN. See, for example, Dranoff et al. (1993) Proc. Natl. Acad. Sci. USA 90:3539-3543; Nohria, A, RH. Rubin (1994) Biotherapy 7:261-269; Wolf et al. (1994) Stem Cells 12:154-168; Kelso, A (1995) Immunol. Today 16(8):374-379; Bentwich et al. (1995) Immunol. Today 16(4):187-191; Garside et al. (1995) Immunol. Today 16(5):220-223; and the references cited in these articles. Additional aspects of the present invention include inhibiting virus replication and virus-mediated carcinogenesis. In one embodiment, inhibition of viral replication is accomplished by cytotoxic T-cells which can destroy cells expressing any viral antigen. Cytotoxic cells are produced by inducing either naive or memory T-cells with antigens expressed in the lymphoid organs. This is a highly advantageous feature of the subject invention because it is well known that viruses, *e.g.,* human papillomavirus, hepatitis C virus, HTLV-1 and human herpesvirus, type 8 (HHV-8) (also called Kaposi's sarcoma herpesvirus (KSHV)), and HIV-1, can mediate cervical and laryngeal cancer, liver cancer, adult T-cell leukemia, and Kaposi's sarcoma, respectively. See, for example, Bedi et al. (1995) Nature Medicine 1:65-68. Inhibition of tumorigenesis, therefore, can be accomplished by killing cells expressing viral antigens independently, whether the virus is directly or indirectly involved in tumor formation.

Another aspect of the invention is a method for generating a safe genetic vaccine against viral infections. This is accomplished as described above by production of attenuated viruses, with or without cytokines, in the lymphoid organs of a naive individual. Efficient antigen presentation in the lymphoid organs results in clonal expansion of antigen-specific T-cells which are necessary to handle a large number of foreign antigens. Some progeny of antigen-responding cells develop antigen-specific memory T-cells which initiate rapidly a larger secondary immune response upon subsequent stimulation.

Thus, the genetic immunization techniques described here can generate from naive T-cells a large number of memory T-cells specific to many or possibly all different viral antigens to avoid the possibility of antigenic escape. This is achieved by the professional APC *(e.g.,* DC) presentation of all viral protein epitopes to different immune cells in the lymphoid organs, as described above. In addition, virus is produced which acts as soluble antigen and infects other cells in the lymphoid organs. Augmentation of the immune response is achieved by reinfection of other cells in the lymphoid organs by the attenuated viruses resulting in secondary antigen presentation. Also, the use of adjuvants (*e.g*., cytokines) or repeated administration of the vaccine can be used to augment the specific immune response.

That such long lasting cross-reactive immunity may be generated has been reported by Selin et al. ([1994] J. Exp. Med. 179:1933-1943). This paper provides evidence that memory cells may be substantially restimulated by viruses that are not serologically cross-reactive. This is true for both T-cell and B-cell responses. It is known that T-cells recognize short peptides of 8-13 amino acids and that they can cross-react to variants of the original peptide, as well as to unrelated MHC/peptide complexes. Therefore, T-cell cross-reactivity may not be limited to related pathogens. Another important feature for cross-reactivity is that memory T-cells are present at the same frequency in both the lymphoid organs and the blood, a property befitting a cell needed for immune surveillance (Lau et al. [1994] Nature 369:648-652). Therefore, unlike naive cells, memory T-cells are exposed to antigens which are not in the lymphoid organs and rapidly respond to infections. The genetic immunization technique of the present invention generates memory T-cells against all viral antigens presented in the correct conformation, not only primarily by dendritic cells but also by secondary infected cells. In infections like HIV or influenza, a small percent of memory cells generated against the conserved or cross-reacting antigens can provide protection against subsequent challenge with another subtype.

Cross-protection between different subtypes of viruses is especially important in the case of HIV and influenza infection, where large numbers of different subtypes can rapidly emerge. Evidence for cross-protection between different subtypes was recently demonstrated by Marlink *et al.,* therefore genetic immunization with HIV-2 may protect against HIV-1 infection (Marlink et al. [1995] Science 265:1587-1590). Moreover, as described, this type of genetic immunization using a whole virus will protect against HIV variants more successfully than subunit vaccines in the art.

The immune response generated by virus (or virus/cytokine) producer cells in the lymphoid organs according to the subject invention is a complete antiviral response. For example, where the producer cells are dendritic cells and the attenuated virus is integrase-negative HIV-1 able to produce proteins in the infected cells, the immune response can occur by the following means:
(a) antigen presenting dendritic cells (primary antigen presenting cells) migrate with the acquired DNA into the lymphoid organs where, upon protein and virus production, they stimulate naive and memory T-cells and provide co-stimulatory signals. Thomson et al. (1995) Transplantation 59:544-550;
(b) immune cells respond to the viral antigens and the free virus produced *in situ* by the dendritic cells. For example, dendritic cells and macrophages present in the lymphoid organs have the ability to present viral antigens to both CD4⁺ and CD8⁺ T-cells;
(c) the anatomical co-location of antigen, antigen presentung cells (APC), and T-cells in lymphoid organs, promotes the initiation of a T-cell response. Mammals use cytotoxic T-cells and natural killer cells to eliminate infected or otherwise compromised cells and tissues. Cytotoxic T-cells recognize peptides presented by MHC class I molecules and natural killer cells look for the absence of MHC-I, a common consequence of virus infection and malignant transformation;
(d) the attenuated virus produced by the dendritic cell may infect other immune cells *(e.g.,* CD4⁺ lymphocytes, dendritic cells, and macrophages) in the lymphoid organs. Viral antigen will also be presented to effector cellos;
(e) the antigen in the lymphoid organs stimulates the production of cytokines. These cytokines have an effector role in T-cell *(e.g.,* Macatonia et al. [1995] J. Immunol. 154:5071-5079) and B-cell mediated immune response, mediate natural immunity (*e.g*., interferon, CD8 inhibitory factor), and regulate activation, growth, and differentiation of lymphocytes and hematopoiesis.
Therefore, production of cytokine(s) together with the attenuated virus can augment and direct the immune response; and
(f) attenuated virus (as extracellular antigen) and CD4⁺ helper lymphocytes can activate macrophages for phagocytosis of the antigen, and activate B-cells for antibody secretion.

Thus, the subject invention is useful in the prevention and treatment of viral infections and other pathological conditions including, but not limited to, cancer. Another aspect includes the combination of DNA encoding the attenuated virus with tumor antigens. Since genetic immunization results in induction of a strong primary and secondary immune response against antigens expressed in the lymphoid tissues, immune response will be also directed against foreign antigens (like tumor antigens) engineered into the Class 4 virus. The materials and methods described herein can be used in the treatment and prevention of a broad range of viral infections. Specifically exemplified herein are materials and methods for the prevention or treatment of pathological conditions of humans or animals caused by retroviruses including HIV and SIV. As used herein; reference to HIV includes HIV-1 and HIV-2, unless the context indicates otherwise.

Following are examples which illustrate procedures for practicing the invention. These examples should not be construed as limiting. All percentages are by weight and all solvent mixture proportions are by volume unless otherwise noted.

### Example 1 - Examples of Infectious but Replication-Incompetent (Class 4) Viruses

The attenuated virus used according to the subject invention must be nonpathogenic. In a preferred embodiment, this lack of pathogenicity will be due to an alteration of the viral genome which destroys its ability to replicate infinitely. Such replication defective viruses are preferably obtained by deletion methods in order to avoid reversion to wild-type virus. Such deletion methods are well known in the art. As used herein, reference to attenuated viruses includes mutant viruses. A comparison and definition of Class 4 viruses are shown in Table 1 and the subsequent text:

| **Tabte 1.** | | | | | | |
|---|---|---|---|---|---|---|
| | Mechanism of attenuation | Virus designation | Replication above threshold | | Disease | |
| | | | In normal host | In co-infected or immuno-compromised host | In normal host | In co-infected or immuno-compromised host |
| 1 | None | Pathogenic | Yes | Yes | Yes | Yes |
| 2 | Loss of pathogenicity | A virulent | (Yes) | (Yes) | No | No |
| 3 | Decreased ability to replicate | Replication-impaired | No | Yes | No | Yes |
| 4 | Infectious but replication-incompetent | Replication-incompetent | No | No | No | No |

The "Mechanisms of attenuation" 1-3 shown in Table 1, based on Baba et al. (1995) Science 270:1220-1221, illustrate the various classes of viral attenuation demonstrated in the art, and the impact of the attenuation on viral ability to cause infection above a threshold level. The present invention introduces Class 4 attenuated viruses, described in detail below. According to the threshold hypothesis, proposed by Baba, retroviral pathogenicity becomes apparent only after the level of viral replication surpasses the threshold level in a given host: The threshold level is defined as that level of infection above which persistent viremia and pathogenicity are exhibited. Figures 3A-3B, based on Baba, illustrates the threshold level concept:

Referring to Table 1 and Figures 3A-3B, Class 1 viruses are unattenuated wild-type viruses, capable of replicating above the threshold level and causing disease in the infected host. Class 2 viruses lack pathogenicity because, although these viruses have the capacity to replicate competently, they are not virulent in the host. Hence the "(Yes)" in the Replication column of Table 1. Class 3 viruses may be replication-impaired by a variety of means, but factors may upregulate viral replication and restore virulence once the level of threshold replication is exceeded. Referring to Figures 3A-3B, Class 3 viruses cause disease in individuals with lower thresholds.

More specifically, infection with a Class 1 or 2 virus (see Table 1) results in persistent viremia (these viruses are always capable of replicating above the threshold), which is a potential danger to the host independent of the pathogenicity of the virus (Figures 3A and 3B). For example, retroviruses integrate in the host genome randomly, therefore the possibility of activating an oncogene through integration is high when there is a high level of replication. And although normal hosts are able to control the replication of Class 3 viruses (Figure 3A), this mechanism is not specific, e.g., for HIV, SIV, or for mutant viruses. For instance, Human Cytomegalovirus (CMV) infection causes a transient viremia in a normal host; however, in patients after bone marrow transplantation or in AIDS (where the immune system is compromised), the threshold of controlling virus replication is decreased (Figure 3B). Consequently, CMV will start to replicate above the threshold and cause serious disease.

Although, in reference to Table 1, it might appear initially that attenuated viruses in Classes 2 and 3 could be suitable for genetic immunization, the opposite is so because these viruses are able to replicate infinitely under optimal conditions (*e.g*., tissue culture, immunodeficient patient, newborn). For example a Class 3 mutant of SIV deleted in the nef and vpr genes causes AIDS in rhesus macaque neonates which have a lower threshold than adults, see Figures 3A-3B. Class 3 viruses are thus relatively unsafe for genetic immunization. Similarly, Class 2 viruses are relatively unsafe for genetic immunization because virulence may be triggered by secondary infection by a virulent virus. For example, while avirulent Rauscher murine leukemia virus (RLV) showed protection against low-dose RLV following inoculation with a Class 2 pharmacologically attenuated RLV, mice co-infected with high-dose virulent mouse leukemia virus developed RLV disease. Class 2 viruses, putatively avirulent, nevertheless do not protect against high-dose viral challenge and also are too dangerous for vaccine purposes because residual virulence may manifest itself. This residual virulence may be triggered by various mechanisms including co-infection, loss of immunocompetence, and aging. Thus, prior art vaccine strategies based on live attenuated virus continue to pose serious dangers.

Class 4 viruses are not described by Baba, but are introduced as part of the invention described herein. One aspect of the claimed invention involves the generation and presentation of Class 4 viruses which are attenuated to remain infectious below the threshold level because they are replication-incompetent. In the Class 4 viruses replication is self limited, meaning that zero to several replication cycles may occur; replication is not infinite and remains below the disease threshold level such that the patient does not contract disease. The Class 4 viruses are also capable of infecting cells, but the infection is incapable of spreading to a significant extent because of the incompetent replication abilities. Class 4 viruses are genetically modified so that they can not survive in nature. Moreover, infection with Class 4 viruses never results in persistent viremia. Class 4 viruses are not able to replicate above the threshold, even if the threshold is the minimum (*e.g*., optimal *in vitro* conditions).

Because of the limited nature of their infection, the Class 4 viruses must be presented to the lymphoid organs in order to effect a protective immunogenic response. Importantly, Class 4 viruses are constructed in such a way that escape from immunity and return to virulence is impossible, unlike Class 2 and Class 3 viruses. Below, we explain in more detail the generation and use of Class 4 viruses. It is preferred that transduced cells produce a high level of Class 4 virus in the lymphoid organs. This may be achieved by the virus' own promoter/enhancer element, or these elements may be replaced by a stronger enhancer (*e.g.,* CMV, SV40 enhancers) to promote gene expression in the transduced cells. The goal is to maximize the amount of virus particles produced in the transduced cells, which results in stronger primary and secondary antigen presentation.

### Example 2 - Integrase-Defective Retroviruses

In the case of retroviruses (*e.g*., HTLV, HIV), the attenuated virus is preferably selected to be integration-defective to reduce pathogenicity and increase safety. The use of such a mutant in the invention is important because, to date, no retrovirus has been shown to be pathogenic in the absence of integration. Sakai et al. (1993) J. Virol. 67(3):1169-1174. An integrase-defective virus may, however, express all viral proteins in native form except the modified integrase (which also can present epitopes). Stevenson et al. (1990) J. Virol. 64(5):2421-2453. Using integrase-defective viruses, the genetic information will eventually be lost by cell division, cell death, and the patient's immune response.

The attenuation must be significant enough to avoid viral mutation or other escape. In particular, a point mutation is sufficient to effectuate the desired loss of replication competence (Cara et al. [1995] Virology 208:242-248), yet this is not preferred because of the risk that the virus could mutate to regenerate a functional peptide. Therefore, preferably, the mutation to the gene would comprise an insertion or deletion of at least three base pairs, and more preferably at least 100 base pairs, as long as the desired level of replication is maintained.

Replication defective viruses harboring a partially or completely destroyed integrase can be constructed several different ways:
(a) In a preferred embodiment, the integrase is 3'-deleted or at least a section near the 3'-end is deleted to place the 3'-end out of reading frame to generate a truncated integrase protein. If the protective immune response requires gene expression also in the infected target cells, an "out of frame" mutation which destroys the 3'-end of the integrase is preferred. This mutation eliminates the active site of the integrase as well as the DNA binding site, and therefore the integrase protein will not bind to the non-integrated DNA, thus allowing gene expression and protein production after infection of the target cells. See Cara et al. (1995) Virology 208:242-248; Stevenson et al. (1990) J. Virol. 64:2421- 2425. The resulting Class 4 virus will not integrate after infection but will be able to express a limited amount of viral proteins in infected cells. Consequently, its replication will be self-limiting: Class 4 viruses stop replicating after several cycles, even under optimal conditions. This strategy generates a strong primary antigen presentation in the transduced cells and also a strong secondary antigen presentation of the infected cells due to viral gene expression, which is optimal for genetic immunization. Similar attenuation can be used with other retroviruses and onco-retroviruses.
(b) Mutation "in frame," preserving the 3'-end of the integrase (LaFemina et al. [1995] J. Virol. 66(12):7414-7419; Sakai et al. (1993) J. Virol. 67(3):1169-1174; Wiskerchen, M., M.A. Muesing (1995) J. Virol. 69:376-386) results in replication- and integration-defective viruses. These Class 4 viruses are able to infect cells but are not able to efficiently express protein after infection of the target cells. Using these viruses for genetic immunization results in an effective primary antigen presentation and also a secondary antigen presentation due to the infection. However, the secondary antigen presentation from the infected cells is expected to be less effective than from cells which are infected and express viral proteins (see above).
(c) 5' deletion of the integrase gene can also be used to produce an integrase- negative retrovirus. However, 5' deletions generate a very unstable protein. Since retroviral integrase is generated from a precursor polyprotein together with reverse transcriptase, it is expected that the precursor will also be instable. This protein instability may reduce the efficacy of virus production by the transduced cells, and consequently influence the efficacy of genetic immunization.
(d) Another way to generate Class 4 HTV-1 which cannot integrate is to delete the vpr gene. Vpr-defective mutants can only replicate in a limited fashion in primary human lymphocytes and not in macrophages because the vpr gene product is important for integration.

Several mutants have been described in the literature (see references cited in Cara et al. [1995] Virology 208:242-248) which are not able to integrate and, therefore, are not able to replicate. Since these viruses are able to infect cells, they may putatively be classified as Class 4 viruses. For example, Cara *et al.* described an integrase negative HIV-1, which has a point mutation and can replicate in a limited way in primary human macrophages but not in T-cells. As described above, however, the preferred embodiment comprises a deletion mutant (not a point mutant that is less safe) which exhibits self limited replication in immune cells (macrophages, dendritic cells or T-cells). This is advantageous over the prior art because the attenuated virus cannot revert to wild-type. In addition, the literature has not reported the growth in dendritic cells of either replication-defective or integrase-defective virus. Similarly, self-limiting replication of a new virus in dendritic cells is a new characteristic to Class 4 viruses for use in this invention. Finally, the literature has not described Class 4 mutant viruses able to generate immune response when they are expressed in the lymphoid organs.

### Example 3 - Ga4-Defective Retroviruses

An example of replication-defective retroviruses which can stimulate a protective immune response according to the subject invention are gag-defective viruses. Gag is processed by the viral protease into several structural proteins. By generating deletion mutations within the capsid domain of the gag gene, mature particle formation is disrupted, but gene expression is preserved. A trans-dominant gag mutant of HIV-1 was described by Trono et al. (1989) Cell 59: 113-120. However, no prior art suggests its use in the present invention. This virus can infect cells but is not able to replicate in the target cells. Moreover, if a wild-type virus infects the mutant virus-containing cells, the mutant virus can block the replication of the wild-type virus. In the case of HIV-1 infected individuals, producing such replication-defective mutant viruses in the lymphoid organs in accordance with the subject invention not only generates an immune response, but also blocks the replication of the wild-type virus. Consequently, the viral load in the lymphoid organs of infected individuals decreases, allowing the immune system to respond.

### Example 4 - Other Class 4 Attenuated Retroviruses

Other Class 4 attenuated HIV viruses which are useful in the present invention include, but are not limited to, env-defective, protease-defective, rev-defective, pol-defective, tat-defective (Beale (1995) Lancet 345: 1318-1319), and gp41 mutant viruses.

Human T-cell lymphotropic virus type-1 (HTLV-1) persists in many years in infected individuals, but unlike with HIV-1, only a minority of them develop disease (adult T-cell leukemia or tropical spastic paraparesis). There is a high degree of intraisolate sequence heterogeneity in HfLV-I, although sequence variation between patients is small compared to HIV-1. The genomic organization of HTLV-1 is similar to HIV, therefore integrase defective HTLV-I can be constructed by any molecular virologist as described for HIV.

Also, other genes such as gag, pol, env, rex, and tax can be modified to generate a Class 4 virus. Although somewhat different, all integrase proteins from retroviruses share similar structures and domains. Therefore, the same considerations illustrated for HIV-1 apply for other retroviruses including not only HIV-2 but also bovine leukemia virus, feline leukemia virus, feline immunodeficiency virus, *etc.*

### Example 5 - Other Class 4 Attenuated Viruses (Reference)

Non-retroviral viruses that can be attenuated to fit the Class 4 model include herpes viruses, influenza viruses and vaccinia viruses (VV). There is a need for the development of Class 4 viruses for immune therapy. For example, efforts to create an attenuated respiratory syncytial virus vaccine have been unsuccessful until recently. Hsu et al. (1995) Vaccine 13(5):509- 515. Although Hsu *et al*. have apparently developed a temperature-sensitive attenuated virus, that virus remains uncharacterized genetically. A better approach would be to design a Class 4 virus as discussed above.

Generally, Class 4 RNA-viruses can be constructed by modification of the polymerase gene. Biswas et al. (1995) J. Virol. 68:1819-1826 demonstrated that viral polymerases contain four conserved motifs critical for primer binding function. Genetic alteration of one or more of these domain renders RNA viruses, including influenza viruses, retroviruses, yeast I-A virus, and poliovirus polymerases impaired; therefore the progeny virus are replication-incompetent.

Herpesviruses that may be attenuated for use in genetic immunization include herpes simplex virus (HSV), pseudorabies virus, and alphaherpesvirus. Specifically, Cheung and coworkers have developed a replication-incompetent virus that is only mildly infectious in swine. The attenuation involved deleting DNA encoding parts of two gene products: early protein (EPO) and large latency transcript (LLT). Cheung et al. (1995) Am. J. Vet. Res. 55(12): 1710-1717.

Similarly, Farrell and coworkers have developed an attenuated live human herpes simplex virus type I capable of only a single round of replication and, therefore, lacking pathogenic potential. This attenuated virus lacks essential glycoprotein H (gH) and can only be propagated in a cell line that provides gH in trans. In order to effect immunization, the virus must be propagated *in vitro* and then inoculated into the subject animal. Farrell et al. (1994) J. Virol. 68:927-932. According to the invention described herein, the DNA encoding either the gH-lacking virus, or preferably a gH-defective virus, can be introduced into cells migrating to the lymphoid organs, where the single round of replication results in a nearly whole virus being presented advantageously to the immune system.

Alternatively, scientists have pursued other replication-defective human herpes simplex type I viruses for vaccine purposes. For example, Morrison et al. (1994) J. Virol. 68(2):689-696 used replication defective (Class 4) HSV bearing mutations in the essential genes encoding infected cell protein 8 (ICP8) or ICP27. Mice were immunized with a high dose of mutant virus by tail-inoculation. Although such viruses have provided immunity in mice, this type of immunization did not provide protection against acute virus replication in the eyes and in the trigeminal ganglion. According to the invention described herein, DNA encoding Class 4 HSV would be introduced into cells migrating to the lymphoid organs, where virus replication and antigen presentation would induce strong antiviral immunity.

Analogous to these examples, based on sequence and functional similarity of herpesviruses, other herpesviruses can be converted to Class 4 viruses and produced in the lymphoid organs. For example, productive infection with HHV-8 (KSHV) was recently demonstrated in Kaposi's sarcoma cells (KS-cells) (classical, post-transplant, and AIDS associated), confirming the epidemiological evidence of the infectious etiology of KS. A genetic immunization, according to the present invention, with Class 4 HHV-8 can be used to confer protection against Kaposi's sarcoma. Another herpesvirus example is the Human Cytomegalovirus (HCMV) infection which causes numerous clinical syndromes in immunocompromised individuals, some which are life threatening. These infections can cross the placenta and infect a fetus *in utero,* resulting in congenital birth defects. Genetic immunization with Class 4 HCMV (which may be made by deletions of genes responsible for viral DNA replication, *e.g.,* ribonucleotide reductase, thymidine-kinase, polymerase) can be used to confer protection to these diseases.

Papillomaviruses are small DNA tumor viruses that are associated with epitheliomas, squamous papillomas, or fibropapillomas depending upon the host and specific papillomavirus type. Despite the encoded oncogen with potent transforming activity, papillomaviruses typically induce discrete epithelial hyperplasias that persist for many months to years. Only occasionally do benign papillomavirus-induced lesions change to progressive growth or convert to malignant neoplasm (Howel et al. [1988] Am. J. Med 85:155-158). Papillomas rarely regress spontaneously (Kreider et al. [1968] Cancer Res. 23:1593-1599), indicating that the immune system can recognize infected cells. Thus, papillomavirus-infected epithelial cells can not induce a sterilizing immune response. The invention described herein overcomes this problem by producing mutant papillomavirus in the lymphoid organs where a potent immune response can be generated. Moreover, recent studies have demonstrated that the product of the virus-encoded E1 gene is essential for viral DNA synthesis in bovine papillomavirus (BPV) (Bonne-Andrea et al. [1995] J. Virol. 69:2341-2350). Thus, a Class 4 BPV can be constructed for genetic immunization by mutation of the E1 gene and deletion of the transforming oncogenes.

Other viruses used in current vaccine strategies with limited effect could gain new efficiency if delivered to the lymphoid organs as described herein. In particular, replication-incompetent poxviruses used as subunit vaccines exhibit little and non-reproducible efficacy. However, the broad host range and the ability to express multiple genes makes vaccinia virus vectors suitable for genetic immunization. Direct inoculation of such vaccinia based vaccines into the skin have not generated a reproducible protective or effective immune response. For example, Gallimore *et al.* reported immunization of monkeys with recombinant vaccinia virus carrying an SN gene ([1995J Nature Medicine 1:1167-1173). Despite triple introduction of high amounts of vaccinia virus by scarification, identically immunized animals made different amounts of CTL responses. Therefore, protective immunization cannot be achieved this way. Introduction of those viruses into cells traveling to the lymphoid organs and the production of these viruses there will increase the potency of the immune response. This recombinant virus can carry any foreign gene, e.g., tumor antigen, to generate, for example, antitumor immune response. The mechanism is similar to that described with HIV, above, but may be stronger because vaccinia infects all types of - cells; therefore it may generate more secondary APCs. Thus, the immune response would be generated against vaccinia as well as against the foreign antigen (or antigens) cloned into the vaccinia virus vector. Non-replicating poxviral vectors have been already created, but not for use in the present invention. See, *e.g.,* Radaelli et al. (1994) Vaccine 12:1110-1117; Tartaglia et al. (1992) Virology 188:217-232; Taylor et at (1991) Vaccine 9:345-358.

Wild type viruses which do not productively infect cells in the lymphoid organs are also Class 4 viruses, if their production is limited in the lymphoid organs. Gene expression from the transduced DNA of these viruses may also generate the required immune response in the absence of pathogenicity. However, for safety reasons, it is suggested that one renders the viruses molecularly replication-incompetent.

### Example 6 -Combination of Class 4 Attenuated Virus with Cytokines

DNA encoding a Class 4 virus may be engineered to express at least one gene encoding an immune modulating molecule in addition to the genetic material encoding the attenuated virus. This creates a "virus/cytokine producer cell." Examples of cytokines which can be used to enhance the immune response or direct a particular immune response include, but are not limited to, IL-1, IL-2, EL-3, IL-4, EL-6, IL-7, IL-12, IL-15, IL-16, TNF, GM-CSF, IFN, and chemokines, such as RANTES and MIPI. See, for example, Dranoff et al. (1993) Proc. Natl. Acad Sci. USA 90:3539-3543; Nohria, A., RH. Rubin (1994) Biotherapy 7:261-269; Wolf et al. (1994) Stem Cells 12: 154-168; Kelso A. (1995) Immunol. Today 16(8):374-379; Bentwich et ai. (1995) Immunol. Today 16(4):187-191; Garside et al. (1995) Immunol. Today 16(5):220-223; Cocchi et al. [1995] Science (Dec. 1995), and the references cited in these articles.

In the case of disease caused by retroviral infection (*e.g*., HIV) it is particularly advantageous for a replication-defective virus to be produced by the virus producer cells in the lymphoid organs. For example, HIV-1, IDV-2, FIV, or *SN*, can infect in the lymphoid organ CD4⁺ T-cells, macrophages and dendritic cells. After internalization and reverse transcription of the Class 4 virus construct of the subject invention, the target lymphoid cells support only minimal replication of the defective virus. Thus, production of intact retroviruses in the cytokine-rich lymphoid organs does induce an immune response against the viral antigens. The exact nature of this immune response can be modulated according to the subject invention based on the nature of the virus-producing cells and the cytokine pattern present in the lymphoid organs. Therefore, the nature of the immune response can be influenced by co-introduction of a cytokine gene into the virus producer cells. Consequently, these cells produce in the lymphoid organs attenuated viruses and cytokines together, thus promoting a specific type of immune response against the virus. For example, the gene encoding IL-12 has been cloned and expressed *in vitro.* Dendritic cells producing IL-12 have been shown to enhance IFN-producing Th1 cells from CD4+ T cells. Macatonia et al. (1995) J. Immunol. 154:5071-5079. Standard techniques may be used to clone the gene for IL-12 into the DNA encoding the Class 4 virus described above, so that cells transduced with this DNA will also express IL-12. The expression of IL-12, along with the Class 4 virus, in the lymphoid organs enhances the subsequent immune response involving Th cells. This technique is applicable with any cloned cytokine gene, as noted above. Moreover, the transfer of genes encoding cytokines into lymphocytes and tumor cells has been proposed (Schmidt-Wolf (1995) Immunol. Today 16(4):173-175), but the combination of the cytokine gene with the Class 4 virus and its production in the lymphoid organ, as contemplated herein, will prove even more advantageous as a method of enhancing genetic immunization.

### Example 7 - Combination of Class 4 Attenuated Virus with Tumor Antigens

As described above, the present invention is useful to generate an antiviral immune response. Another aspect of the subject invention includes the combination of DNA encoding the attenuated virus with tumor antigens. Since genetic immunization results in induction of a strong immune response against antigens expressed in the lymphoid tissues, the immune response can also be directed against foreign antigens engineered into a Class 4 virus. All tumor cells express genes, the products of which are required for malignant transformation. Even after transformation, tumor formation is often the result of an inefficient immune response. It is also well known that an effective antitumor immune response can result in tumor regression. Tumor cells which express antigens that induce an immune response in the host have been demonstrated in both animal models and human cancer patients. See generally Abbas *el al., supra* at pp. 357-375. Therefore, Class 4 viruses can be used to generate an antiviral immune response which in turn generates anticancer immunization. Examples of such antigens include MAGE-1, exhibited in 50 percent of melanomas and 25 percent of human breast carcinomas; p21ras proteins with point mutation at position 12, exhibited in 10 percent of human carcinomas; p21 product of bcl/abl rearrangements, exhibited in chronic myelogenous leukemia; Human papillomavirus E6 and E7 gene products, exhibited in human cervical carcinoma; and EBV EBNA-1 gene product exhibited in Burkitt's lymphoma and nasopharyngeal carcinoma. Two more-thoroughly characterized human cancer-related antigens are alpha-fetoprotein and carcinoembryonic antigen. Recently, Grabbe *et al.* have reported that the differential presentation of tumor antigens by antigen-presenting cells may be critical for the effectiveness of tumor immune responses. (Grabbe et al. [1995] Immunol. Today 16(3): 117-121.)

In a preferred embodiment of this invention, dendritic cells transduced by a DNA encoding a Class 4 virus effectively elicit an immune response. The DNA encoding the Class 4 virus can be engineered to also express one or more tumor antigens. Accordingly, cells expressing and presenting antigens in the lymphoid organs efficiently elicit the host's immune response against tumors bearing that antigen(s) in addition to the immune response against the attenuated virus. Cytokine genes also cloned into Class 4 viruses with the tumor antigen can influence and augment the immune response. One example, not within the scope of the invention, for Class 4 viruses which can harbor tumor antigens is the recombinant vaccinia virus. This virus is easy to manipulate to efficiently express foreign genes; also its broad tropism makes it possible to generate not only primary antigen presenting cells but also secondary antigen presenting cells. Another example is the use of integrase negative HIV. In this virus, nef is an early highly expressed protein. Introducing a tumor antigen in the nefopen reading frame and/or in the deleted integrase can confer not only antiviral but also antitumor immune responses.

### Example 8 - Transduced Cells

The virus producer cells of the present invention are preferably transduced cells. Transduced cells carry the DNA encoding the Class 4 attenuated virus, and optionally in addition to the viral DNA, genetic material encoding cytokine(s) and/or tumor antigen(s), into the lymphoid organs, where the DNA encoding the attenuated virus, cytokine, and/or tumor antigen is expressed. The cytokine and tumor antigen encoding DNA are preferably introduced to the virus to generate secondary antigen presenting or cytokine producing cells. However, this is not necessary. Cytokine or tumor antigen DNA can also be introduced into the virus producer cells on a separate, independent plasmid. Also, virus producer cells can be mixed with cytokine producer cells to achieve the same augmentation of the immune response.

In a preferred embodiment of the subject invention, the cells which are transduced with any of the aforementioned attenuated viruses, including, for example, integrase-defective or replication-defective viruses, are the cells of lymphoid organs or are cells which preferentially migrate to the lymphoid organs. Transduction of these cells is particularly advantageous because the lymphoid organs are the primary site of the induction of a host's natural defenses against viral infections. In a particularly preferred embodiment, the transduced cells are dendritic cells. However, several different types of cells can be used as virus producer cells in the lymphoid tissue. The cells that can be used as virus producer cells are discussed in more detail in the Examples which follow.

### Example 9 - Autologous or Syngenic Dendritic or Langerhans Cells

Class 4 virus producer cells are preferably produced by introducing DNA encoding an attenuated virus to dendritic cells (abundant in the lymph nodes and spleen) or Langerhans cells (found in the epidermis) which are bone marrow-derived and extremely efficient at presenting antigens to CD4+ helper T-cells, CD8⁺ cytotoxic T-cells, and naive T-cells. Grabbe et al. (1995) Immunol. Today 16(3):117-121. Langerhans cells are capable of migrating from the skin to the lymphoid organs. Abbas *et al., supra* at p. 124. Moreover, Thomson et al. ([1995] Transplantation 59:544-551) have shown that *in vitro*-cultured dendritic cells are homing to the draining lymph nodes and spleen, where they persist at least two months. Dendritic cells are, indeed, "professional antigen presenting cells." Additionally, foreign protein presented by dendritic cells can generate a CTL response (Rouse et al. [1994] J. Virol. 68:5685-5689), which is advantageous for the ultimate complete elimination of infected or viral antigen-producing cells.

Dendritic cells can be isolated from bone marrow, peripheral blood, umbilical cord blood, and other organs utilizing techniques known to those skilled in this art. A simple procedure described to generate dendritic cells has been recently described using granulocyte macrophage colony stimulating factor (GM-CSF) plus IL-4 (Romani et al. [1994] J. Exp. Med. 180:83-93; Sallusto et al. [1994] J. Exp. Med 179:1109-1118).

Thus, a preferred embodiment of this invention employs dendritic cells to carry the Class 4 attenuated viruses to the lymphoid organs. These cells can be isolated, transduced with DNA encoding an attenuated virus, and reintroduced to the host. DNA encoding attenuated virus such as the 3'-deleted integrase defective virus described above, can be introduced into Dendritic cells by the processes outlined in Examples 13 and 14.

### Example 10 - Allogenic Dendritic Cells

In accordance with the present invention, allogenic dendritic cells isolated, for example, from cord blood, can also be transduced to create virus producer cells. This allows antigenic producer-cell transplantation in the absence of graft-versus-host disease. Allogenic cells are easily obtained through practices well known to those of ordinary skill in the art. DNA encoding the attenuated virus is introduced into allogenic cells by the methods described below in Examples 13 and 14.

### Example 11 - Fibroblasts

Fibroblasts can also be transduced with a Class 4 attenuated virus of the present invention to create virus producer cells. It is known in the art that fibroblasts can be used to induce T-cell response because they carry and efficiently present the antigen to the lymphoid organs (Kundig et al. [1995] Science 268:1343-1347). Fibroblasts are easily obtained via practices known to those of ordinary skill in the art and are transduced by the methods described below in Examples 13 and 14.

### Example 12 - Allogenic or Xenogenic Cells

In accordance with the subject invention, transduced cells carry Class 4 DNA to lymphoid organs and produce virus. The free virus in the lymphoid organ stimulates immune cells for antigen presentation and immune response. Consequently, the transduced cells are recognized by the immune system as infected cells and are eliminated, regardless of whether the cells are autologous or allogenic. The difference may be the speed of elimination. In case of efficient virus production and antigen presentation transduced cells may induce sufficient immune response in a shorter period of time. If the transduced cell itself is unable to act as the primary APC, the produced virus can function as a free antigen, and the infected cells can function as secondary APCs. Large amounts of either allogenic or even xenogenic transduced cells may be produced, and aliquots can be used for genetic immunization for all individuals.

It should be apparent to those skilled in the art that in addition to those discussed above, any other cells able to produce virus or virus/cytokine(s)/tumor antigen(s) in the lymphoid organs can also be used as virus producer cells to introduce Class 4 attenuated viruses directly to the typhoid organs to achieve the advantages of the present invention as herein described.

### Example 13 - In vitro Transduction and Administration of Transduced Cells

Genetic material is introduced into the appropriate cells to convert them to virus or virus/cytokine producer cells. The appropriate cells are obtained through any variety of methods known to those of ordinary skill in the art. The cells may be obtained from the patient to be treated, as an autologous source. Presentation of antigens in association with MHC and co-stimulatory molecules results in enhanced cell mediated response. Cells from different individuals or different species may be selected for transduction into an unrelated patient because Class 4 virus is produced from transduced cells which can infect the patient's host cells which then present the antigen in association with the host MHC and co-stimulatory molecules. Therefore these xenogenic infected cells can also function to carry the viral DNA to the lymphoid tissue.

*In vitro* transfer of DNA encoding Class 4 viruses can be performed by using, for example, infection, electroporation, liposomes, virosomes, DNA-polylysine-adenovirus complexes, or polyethylenimine. These methods are well known to those skilled in the art. For example, methods for using liposomes to deliver genetic material to specific cells have been described in detail in WO 92/06677 (Schreier et al. [1992]). Alternatively, viruses (*e.g*., vaccinia or pox viruses) capable of abortive infection (more than one cycle) can be used to effectively transduce DNA in the cell and produce Class 4 viruses.

In accordance with the present invention, virus producer cells, such as transduced cells, may be administered to the human or animal subject by:
(a) Intrasplenic injection. This is preferred because it results in the highest probability of the transduced cell reaching the lymphoid organs.
(b) Injection directly into the lymphatic system.
(c) Injection directly into the tonsils is also preferred because it is an accessible lymphatic organ.
(d) Intravenous injection. The dose depends on the amount of virus produced by the virus producer cells. Repeated administration of virus producer cells can be used to augment the immune response.
(e) Intraperitoneal injection.
(f) Intradermal injection.
(g) Intramuscular injection.

In practice, the optimal number of cells to be injected and the repeated injection must be established on a per-patient basis, based on the generated immune response. This determination can readily be made by a person skilled in the art having the benefit of the subject disclosure.

### Examples 14 -In vivo Transduction

In accordance with the present invention, DNA encoding a Class 4 attenuated virus, with or without associated tumor antigen or cytokine genes, may be administered directly to the cells for transduction and migration to the lymphoid system by:
(a) Direct DNA injection. After intramuscular, intrasplenic, intratonsillar or intradermal injection of the plasmid construct, the DNA is picked up by cells which become virus or virus/cytokine(s) producer cells in the lymphoid organs. D. Weiner (1995) Aids Research & Human Retroviruses 11(1):S136) has demonstrated that DNA injected intramuscularly is expressed and generates an immune response. DNA so incorporated by dendritic cells present near the injection site may be carried to and expressed in the lymphoid organ. Direct DNA injection in accordance with the present invention preferably is made into sites loaded with dendritic cells, *e.g*., the tonsils or spleen, to achieve optimal antigen presentation.
(b) liposomes can be used to deliver DNA to dendritic cells. Intradermal, intrasplenic, intratonsillar, intramuscular and intravenous injections are preferred.
(c) Direct intrasplenic injection of Class 4 pox viruses, which can abortively infect human cells. DNA can be delivered *in vivo* directly to antigen presenting cells by infection and virus particles will be produced in the lymphoid organs.
(d) Rectal or vaginal suppositories carrying, for example, DNA, liposomes, or virosomes may be a particularly advantageous method of delivering DNA encoding Class 4 constructs to cells migrating to the lymphoid organs. Moreover, suppositories avoid the need for sterile needles or medical facilities, and provide antiviral therapies otherwise lacking in developing countries. See, *e.g*., Marlink et a/. (1994) Science 265: 1587-1590.

### Example 15 - Genetic Immunization for Prevention and Treatment of Diseases

Genetic immunization according to the subject invention results in induction of a strong immune response against antigens produced in the lymphoid organs; consequently, genetic immunization according to the subject invention is useful for prevention and treatment of diseases like viral infections and cancer. Additionally, attenuated, replication-defective, and integration defective viruses used for genetic immunization are highly advantageous because they cannot establish infection, cannot induce oncogene expression or gene disruption as result of integration, and do not influence cells responsible for reproduction.

In the case of an epidemic like influenza or HIV, or for individuals with a high risk of cancer (*e.g*., breast cancer), the use of genetic immunization as prevention is particularly appropriate. In the case of viral infections the genetic immunization preferably generates long-lasting and cross-reactive immunity. This is not possible with present vaccine candidates because subunit vaccines often do not present antigens in the correct place or in the correct conformation, and the likelihood of inducing cross-reactive memory T-cells is small. Although live attenuated viral vaccines have provided cross-protection, that protection was only generated several months after immunization.

The invention described herein solves major problems in vaccine development by presenting antigens in correct conformation in the place where the immune response is generated. Consequently, the immune response is long-lasting and cross-protective. Moreover, since an attenuated virus is produced where the immune cells are concentrated, infection of bystander cells results in antigen presentation which leads not only to induction of humoral and cellular immune responses but also induction of natural immunity (*e.g*., natural killer cells and macrophages) and production of soluble factors (*e.g*., cytokines, interferon, chemokines). Memory T-cells, other responding cells and soluble factors continuously circulate in the blood and reach the skin and the mucosa, where they generate a rapid immune response to viral or tumor antigens. Moreover, genetic immunization against HIV-1 can result in a long term cross-protective immunity. Similarly, attenuated viruses can be used for vaccination against other retrovirus-induced diseases because retroviruses cannot replicate in the absence of integration. Integrase defective retroviruses can immunize cats against FN or bovines against BLV.

The genetic immunization techniques of the present invention can also induce an immune response in cases where the pathogen or disease is already present and is especially beneficial for treatment when the immune response induced by a pathogen or oncogen is not adequate to eliminate infected cells or tumor cells. In the case of HN infection, the therapeutic effect of genetic immunization depends on how fast the response is carried out after infection. Long term non-progressors, characterized by low virus burden and intact lymphoid organs, may clear the virus after genetic immunization. In contrast, patients characterized by high viral load and destroyed lymphoid organs may not develop therapeutic immunity. These patients' bodies should first be repopulated by cells genetically resistant to infection, which can be achieved by introducing an antiviral gene into stem cells and/or peripheral cells (Lisziewicz et al. [1995] J. Virol. 69:206-212*).* If the immune system is able to regenerate it will automatically eliminate the residual viruses.

The genetic immunization techniques of the present invention can also be used as immunotherapy for tumors. Current strategies are aimed to create a cytokine-rich environment by transfecting tumor cells with cytokines or converting the tumor cells to antigen presenting cells by transfecting them with co-stimulatory molecules. Kundig et al. ([1995] Science 268: 1343-1346) suggested that tumor cells can be delivered into the lymphoid organs, which are naturally cytokine rich, to induce a protective immune response. The subject invention provides for genetic immunization against tumors. One application is for the treatment or prevention of tumors generated by a viral infection (directly or indirectly). In these cases, viruses can be genetically modified to become Class 4 and genetic immunization performed according to the present invention.

The subject invention can also be used for the prevention and/or treatment of tumors which are not a result of a viral infection. All tumor cells express genes, those products required for malignant transformation. In many instances, tumor-causing genes have already been identified. For example, MAGE-1 (melanoma antigen-1) is expressed in up to 50 percent of melanomas and percent of breast carcinomas, but it is not detectable on normal tissues. This gene can be inserted into a Class 4 virus (*e.g*., HIV) and transduced into cells where an immune response is generated against the tumor antigen and the Class 4 virus. Another method used to generate an immune response against tumor antigens is to transduce into the cells a plasmid expressing only the tumor antigen; however, in this case, a secondary immune response (due to infection of bystander cells) cannot be generated against the tumor antigen. The cytotoxic T-cell response generated by this genetic immunization will eliminate the tumor cells and the memory cells will be responsible for immune surveillance against this tumor.

Any Class 4 virus can be used according to the subject invention for antitumor genetic immunization. It is preferred that the Class 4 virus has tropism to infect the immune cells in the lymphoid organs to generate the secondary antigen presenting cells. Generation of an immune response against several tumor antigens has great advantages in cases where one kind of tumor can express different tumor antigens (for example, only 50 percent of melanoma express MAGE-1).

### Example 16 - Genetic Immunization against HN

Following is a step-by-step description ofone embodiment for carrying out the methods of the subject invention.

### 1. Preparation ofDNA encoding integration defective HIV-I and SIV

(a) Deletions into the integrase gene are introduced by random mutagenesis in order to truncate the integrase in random fashion. *In vitro* characterization of the mutant viruses can be used to confirm that the progeny of the virus is Class 4 and is capable of gene expression and antigen presentation after infection of target cells. Generating an "out of frame" deletion in the central part of the integrase gene is preferred. Figures 2A--2B illustrate the wild-type HIV genome, the particulars of the integrase protein, and an example of the attenuated integrase gene that generates a Class 4 virus. Specifically, this mutation results in a truncated integrase protein which starts at the 5'-end as the wild-type and stops at the first stop codon after the deletion. The mutant protein can be, for example, about half the size of the wild-type integrase.
(b) Targeted deletion within the integrase gene can be generated by PCR (in the presence of a proofreading enzyme). Plasmid DNA encoding an HIV or SIV provirus can be used as a template. Primers are designed to anneal to the integrase gene at the site of the desired deletion(s). The deletion can start 5' from the active site of the integrase and finish at the 3'-end of the integrase. The generated PCR product is a double stranded DNA, which is ligated to obtain a circular plasmid with the deleted integrase gene. DNA is amplified by PCR or transformation into a microorganism such as *E. coli* or yeast. Using this strategy, the 3'-end of the integrase gene can be eliminated. This is a preferred strategy because (a) epitopes from the 5'-end of the integrase are presented from transduced or infected cells, (b) destruction of the central part of the integrase results in deletion of the active site of the protein (maximizing safety because of the lack of integration), and
(c) eliminating the 3'- end of the molecule decreases the binding of the integrase to the unintegrated DNA. Since this protein-DNA interaction inhibits gene expression, maximum efficiency of gene expression in the infected cells is achieved by truncation of the integrase protein at the 3'-end. Similar truncation of the integrase was already demonstrated (Cara et al. (1995) Virology 208:242-248), however, the Cara mutation was a point mutation which is less safe for the genetic immunization of humans. In addition, Cara and other work with integrase-negative viruses have only analyzed the virus production in lymphocytes and macrophages, but have not characterized the antigen presenting properties of the infected cells, which is a key element for genetic immunization.

### 2. In vitro characterization of integration defective viruses and selection of Class 4 viruses for genetic immunization.

(a) Characterization of Class 4 integrase defective viruses. In one embodiment, integrase-negative virus particles are produced after transfection (CAPO₄ procedure) of 293, HeLa or HeLa-Tat cells lines. The amount of viral particles can be determined by p24 or, in the case of SIV, p27 antigen capture assay (Immimotech). These viruses are then used for infection of activated (PHA + IL-2) primary human peripheral blood mononuclear cells (PBMC), primary CD4⁺ T-cells, primary macrophages and/or dendritic cells cultured under optimal conditions for HIV/SIV infection. Virus production and spread can be monitored by antigen capture assay and PCR. Antigen presentation can be determined by a T-cell proliferation assay. These experiments are designed to ensure the optimal conditions for virus replication. Class 4 viruses will either not establish infection or the replication will terminate after several cycles. Preferably, mutant viruses which productively infect cells under these optimal conditions are not further considered for genetic immunization. It can also be determined whether these infected cells can present antigens to CD8⁺ and CD4⁺ HIV-1 specific T-cells and for naive T-cells. If the virus can express genes in the absence of integration in the infected cells, it will activate HIV-1 specific memory cells. Infected dendritic cells will present antigens and activate CD8⁺ naive and memory cells and also present viral antigens for CD4⁺ T-cells. The optimal integration defective virus will infect CD4⁺ T-cells, macrophages and dendritic cells and express viral genes in the target cells for antigen presentation.
(b) Transduction of human dendritic cells. Human dendritic cells can be isolated from peripheral blood, bone marrow and cord blood. The cells can be cultured with GM-CSF and IL-4. DNA encoding the integrase negative HIV and SIV can then be transduced into these cells. Electroporation, infection, liposomes, virosomes, and/or polyethylenimine-mediated gene transfers can be applied, and optimal conditions for transduction are established using standard techniques. After transduction, virus production can be monitored by p24 antigen capture assay and the number of virus producing cells monitored by immunofluorescence assay. As described above, antigen presentation to memory and naive T-cells can be assayed. Strong activation of naive and memory T-cells by transduced dendritic cells results.
(c) Transduction of lymphocytes. Primary CD4⁺ lymphocytes are isolated from normal human donors, activated with PHA and cultured with IL-2. Integrase-negative HIV DNA is then introduced (*e.g*., electroporation or other methods) and virus production monitored by p24 antigen capture assay. This experiment can be used to further ensure that integrase-defective viruses cannot establish productive infection. Similar experiments can be done with SIV using monkey cells.
(d) Transduction of murine fibroblasts and dendritic cells. Integrase-negative HIV-1 DNA can be transduced into dendritic cells and fibroblast of murine origin. Virus production is monitored by p24 antigen capture assay. This experiment can be used to determine whether murine cells are able to express a particular Class 4 virus.
(e) Evaluation of immune response in human tonsil histoculture. Human tonsil cultures may be used as a model for studying the human immune response. This is beneficial in the present invention if animal models do not fully mimic the characteristic pathology of HIV infection. A tissue culture method was recently developed (Glushakova et al. [1995] Nature Medicine 1:1320-1322) which supports HIV-1 replication according to the current invention. Integrase-negative HIV-1 DNA can be transduced into autologous dendritic cells isolated from peripheral blood as described above. Varying amounts of these cells are then microinjected into the human tonsil tissue blocks. Induction of CTL and antibody responses are monitored. In subsequent experiments, tonsil cultures (injected with dendritic cells) can be challenged by infection with different types of HIV and the inhibition of virus replication measured by antigen capture assay. Cross-protection of different subtypes is easily studied in this model because of the generation of a strong antiviral immune response in this system. Similar experiments are performed in tonsil cultures isolated from infected individuals, preferably children.

### 3. in vivo (murine model) testing of genetic immunization. If the experiment described in 2(d), above, demonstrates virus production by transduced murine dendritic cells or fibroblasts, the following experiments can be performed in mice. (Alternatively, human CD4 transgenic mice may be used instead of normal mice. In this model HIV-1 cannot establish productive infection, however it may have the advantage that the mutant virus is able to enter into bystander immune cells.)

Murine model: Tumor cells expressing HIV-1 proteins (*e.g*., gp 120) can be generated by transfection and *in vitro* selection. These cells are then inoculated into mice which produce tumors. Induction of immune surveillance via both the MHC class I and class II pathways is accomplished by genetic immunization and will protect animals against tumor challenge. This murine model also tests for genetic immunization against tumors because integration defective HIV-1 carries as part of the viral genome gp 120 which, in this model, is a tumor antigen. This model differs from the previously described models because the transduced cells produce a free virus which can be captured by other cells (*e.g*., by phagocytosis) in contrast to only expressing an antigen. Other tumor models can also be used in conjunction with expressing the corresponding tumor antigen.
(a) To assess the efficacy of genetic immunization, different doses (10 to 10') of murine dendritic cells cultured with GM-CSF and IL-4 and transduced with integrase defective HIV-1 can be injected intravenously, intradermally, intramuscularly, intraperitoneally, and into the spleen of syngenic mice. This allows examination of whether genetic immunization with transduced dendritic cells induces a CD8⁺ CTL response against gp120 *in vivo* and *in vitro.* Mice are inoculated with the gp120 expressing tumor cells. Development of tumors is subsequently monitored for up to, for example, 60 days. The specific cytotoxicity of gp 120 is also measured after *in vitro* restimulation of spleen cells according to Zinkemagel et al. ([1985] J. Exp. Med. 162: 2125). This experiment can also be used to evaluate the role of the site of the injection in a particular system.
(b) The experiment described in (a) may be repeated in CD4 transgenic mice. CD4 cells of these mice can be infected by HIV, therefore comparison of genetic immunization between normal and CD4 transgenic mice can be used to evaluate the role of the secondary antigen presenting cells in the generation of immune responses.
(c) Murine fibroblasts (*e.g*., fibroblastoma cell line) transduced with integrase-defective HIV-1 can be injected as described above to determine the efficiency of the immune response of genetic immunization with "non-professional" antigen presenting cells in a particular system.
(d) Experiment (a) can be repeated in allogenic recipient cells to assess whether autologous or syngenic cells are best for a particular genetic immunization. Since HIV does not infect murine cells, secondary antigen presenting cells will be not generated in mice. Therefore, cross-priming with host MHC may be required in mice, but not in monkeys or humans. Alternatively, the produced virus may be processed by the allogenic recipient professional antigen presenting cells (even in the absence of infection), which may also result in rejection of tumor and CTL response to viral antigens.
(e) Direct injection (intravenous, intrasplenic, intradermal, intramuscular, intraperitoneal) of different amounts (1-40 µg) of DNA encoding an integrase defective HIV may be used instead of injection of the transduced cells for procedures described above to assess the efficiency of *in vivo* introduction of DNA and whether this method will result in transduction of antigen presenting cells, generation of CTL responses, and elimination of tumor cells in a particular embodiment.
(f) Different amounts (1-40 µg) of DNA encoding an integrase-defective HIV-1 can be encapsulated into liposomes and virosomes for injection (intravenal, intrasplenic, intradermal, intramuscular, intraperitoneal) into a mouse or other host as described above. These procedures help to assess the efficiency of these gene delivery systems can transduce immune cells *in vivo,* and whether *in vivo* transduction results in an effective CTL response capable of eliminating gp120-expressing tumor cells.
(g) When one of the above procedures results in tumor rejection and generation of a CTL response to the produced virus, the murine model may then be readily used for testing of rectal and vaginal suppositories carrying the integrase deficient HIV DNA encapsulated into liposomes or virosomes.
(h) Replication defective vaccinia viruses are able to infect murine cells because of their broad host range. Dendritic cells can be infected with gp 120-expressing vaccinia virus which abortively infects cells (instead of transduction with DNA). As infected cells can produce infectious virus particles (monitored *in vitro* by titration on a susceptible cell line), these cells are used to determine the efficiency of genetic immunization by procedures described above.
(i) Once replication-impaired vaccinia (or other pox) is shown to infect murine dendritic cells *in vitro* and produce infectious particles, *in vivo* infection into the spleen can then be performed using different viral doses. Spleens of the sacrificed animals are monitored for vaccinia production after *in vivo* infection. Animals can also be challenged with tumor cells. Tumor development and antitumor CTL activity are subsequently measured.

Murine Moloney Leukemia Virus (MMLV) can efficiently infect murine cells and cause leukemia in the murine model. Integrase-defective MMLV can be constructed as described for HIV-1 and used for genetic immunization in procedures described above. The advantage of this model is that mice are the natural hosts of MMLV (as are humans for HIV or monkeys for SIV) and inhibition of virus replication can be measured instead of tumor formation. Also, integrase-negative Feline Immunodeficiency Virus may be used in cats as an animal model; however, to assess the antiviral activity of genetic immunization, we prefer the use of non-human primates because the results can be applied most readily to humans.

### 4. In vivo (non-human primate model) testing of genetic immunization. Macaques infected with SIV develop AIDS within two years, and the disease progression is very similar to HIV in humans. Using this animal model, genetic immunization can be tested as prevention as well as therapy. Integration-defective SIV is expressed effectively in macaque cells (gene expression is properly activated by tat), and the produced virus in the lymphoid organs can infect other immune cells and therefore generate secondary antigen presenting cells. This is an excellent model for testing the efficacy of genetic immunization.

(a) Autologous dendritic cells cultured with GM-C SF and IL-4 can be transduced with integration defective SIV. After intrasplenic injection of different amounts of transduced dendritic cells into naive animals, monkeys are challenged with infectious doses of different SIV subtypes. Humoral and cellular immune response and cytokine production before and after challenge are determined. After challenge, samples from peripheral blood and lymph nodes can be analyzed by RT-PCR to detect viral load. This procedure determines the optimal amount of dendritic cells that needs to be injected to achieve protective and cross-reactive immunity.
(b) The level of cross-protection between different lentiviruses can readily be determined for a particular system. Animals can be genetically immunized with a replication- and integrase-defective HIV-1. Subsequently, immunized animals are challenged with pathogenic SIV.
(c) The efficacy of a particular genetic immunization in SIV-infected monkeys can be studied as follows. Autologous dendritic cells cultured with GM-CSF and IL-4 can be transduced with integration-defective SIV. SIV-infected animals receive intrasplenic injections of transduced dendritic cells shortly (1 week to 6 months) after SIV challenge. Humoral and cellular immune responses and cytokine production before and after genetic immunization are determined. After challenge, samples from peripheral blood and lymph nodes are analyzed by RT-PCR to monitor viral load. This procedure helps to assess the efficacy of a particular genetic immunization as immunotherapy against SIV infection.

### Example 17 - Cloning of Integrase-Defective SIV for Evaluation of the Genetic Immunization Strategy in Non-Human Primates

Using the two primers: 
FL5: 5'-CTA CTA TGG TAC CCC AAA GGT GTG CTC-3'     (SEQ ID NO. 1)
(2x stop)
 and
FL6: 5'-TGA ATT TTA AAA GAA GGG GAG-3', (SEQ ID NO. 2)
long PCR will generate the plasmid shown in Figure 4 harboring a deletion in the integrase gene between nucleotides 6115 and 6257. We have also included two in-frame stop codons to ensure the generation of the truncated integrase protein (two ensures safety). The parent plasmid, p239SpSp5', can be obtained through the AIDS Research and Reference Reagent Program, Division of AIDS, NIAID, NIH, from Dr. Donald Desrosiers. This plasmid can be used as described by Ogden Bio-Serviecs Corp., 685 Lofstrand Lane, Rockville, MD 20850, "Special Characteristics," to generate both lymphotropic and macrophage-tropic SIV. For genetic immunization, the macrophage-tropic virus is preferred because of its broader tropism: it can infect both lymphocytes and macrophages.

This is only one strategy to be used as an example. Other mutants can be characterized to obtain a phenotype which is capable of only limited replication cycles in macrophages.

It should be understood that the examples and embodiments described herein are for illustrative purposes only, and that various modifications or changes in light thereof will be suggested to persons skilled in the art and are to be included within the spirit and purview of this application and the scope of the appended claims.

## Claims

1. Use of an immunogenic genetic construct which expresses a replication-incompetent lentivirus in a host's lymphoid organ for the manufacture of a medicament for raising an immune response in the host to the lentivirus.

2. Use of a dendritic cell transduced with an immunogenic genetic construct which expresses a replication-incompetent lentivirus in a host's lymphoid organ for the manufacture of a medicament for raising an immune response in the host to the lentivirus.

3. Use according to claim 1 or claim 2, wherein the virus is integration-defective.

4. Use according to claim 3, wherein the virus has an integrase gene with a mutation comprising an insertion or deletion of at least three base pairs.

5. Use according to claim 4, wherein the integrase gene is mutated in the active site and the DNA binding site.

6. Use according to claim 1 or 2, wherein the virus has a mutation in the gag gene.

7. Use according to claim 1 or 2, wherein the virus is selected from HIV-1, HIV-2, SIV and FIV.

8. Use according to any preceding claim, wherein the genetic construct is capable of transducing a cell, or the cell is transduced, to express a cytokine, to augment or direct the host's immune response.

9. Use according to claim 8, wherein the cytokine is selected from IL-1, IL-2, IL-3, IL-4, IL-6, IL-7, IL-12, IL-15, IL-16, TNF, GM-CSF, IFN, and chemokines of RANTES, M1P1.

10. Use according to claim 8 or claim 9, wherein the expression of the cytokine stimulates a Th1 or Th2 type immune response, a natural killer cell response, a cytotoxic T-cell response or a B-cell response.

11. A pharmaceutical composition comprising a dendritic cell which has been transduced with an immunogenic genetic construct which comprises plasmid DNA that expresses a replication-incompetent lentivirus virus, for use in a method of immunisation.

12. A composition according to claim 11, wherein the virus is as defined in any of claims 3-5.

13. A composition according to claim 11 or claim 12, wherein the virus is as defined in claim 6 or 7.

14. A composition according to any of claims 11 to 13, wherein the cell is transduced to express a cytokine as defined in any of claims 8 to 10.

## Patentansprüche

1. Verwendung eines immunogenen genetischen Konstrukts, das ein replikationsinkompetentes Lentivirus in einem lymphoiden Organ eines Wirts exprimiert, zur Herstellung eines Medikaments zum Steigern einer Immunantwort im Wirt des Lentivirus.

2. Verwendung einer dendritischen Zelle, die mit einem immunogenen genetischen Konstrukt transduziert ist, das ein replikationsinkompetentes Lentivirus in einem lymphoiden Organ eines Wirts exprimiert, zur Herstellung eines Medikaments zum Steigern einer Immunantwort im Wirt des Lentivirus.

3. Verwendung nach Anspruch 1 oder 2, wobei das Virus integrationsdefekt ist.

4. Verwendung nach Anspruch 3, wobei das Virus ein Integrasegen mit einer Mutation umfassend ein Einfügen oder einen Verlust von mindestens drei Basenpaaren aufweist.

5. Verwendung nach Anspruch 4, wobei das Integrasegen an der aktiven Stelle und der DNA-bindenden Stelle mutiert ist.

6. Verwendung nach Anspruch 1 oder 2, wobei das Virus eine Mutation im gag-Gen aufweist.

7. Verwendung nach Anspruch 1 oder 2, wobei das Virus aus HIV-1, HIV-2, SIV und FIV ausgewählt ist.

8. Verwendung nach einem vorhergehenden Anspruch, wobei das genetische Konstrukt in der Lage ist, eine Zelle zu transduzieren, oder die Zelle transduziert wird, um ein Zytokin zu exprimieren, um die Immunantwort des Wirts zu steigern oder zu lenken.

9. Verwendung nach Anspruch 8, wobei das Zytokin aus IL-1, IL-2, IL-3, IL-4, IL-6, IL-7, IL-12, IL-15, IL-16, TNF, GM-CSF, IFN und Chemokinen von RANTES, M1P1 ausgewählt ist.

10. Verwendung nach Anspruch 8 oder 9, wobei die Expression des Zytokins eine Immunantwort vom Typ Th1 oder Th2, eine natürliche Killerzellenantwort, eine zytotoxische T-Zellenantwort oder eine B-Zellenantwort stimuliert.

11. Pharmazeutische Zusammensetzung umfassend eine dendritische Zelle, die mit einem immunogenen genetischen Konstrukt transduziert worden ist, das Plasmid-DNA umfasst, die ein replikationsinkompetentes Lentivirus-Virus umfasst, zur Verwendung bei einem Immunisationsverfahren

12. Zusammensetzung nach Anspruch 11, wobei das Virus wie in einem der Ansprüche 3-5 definiert ist.

13. Zusammensetzung nach Anspruch 11 oder 12, wobei das Virus wie in Anspruch 6 oder 7 definiert ist.

14. Zusammensetzung nach einem der Ansprüche 11 bis 13, wobei die Zelle transduziert wird, um ein Zytokin wie in einem der Ansprüche 8 bis 10 definiert zu exprimieren.

## Revendications

1. Utilisation d'une construction génique immunogène, qui exprime un lentivirus incompétent pour la réplication, dans un organe lymphoïde d'un hôte, pour la préparation d'un médicament pour amplifier une réponse immune dans l'hôte du lentivirus.

2. Utilisation d'une cellule dendritique, transduite avec une construction génétique immunogène, qui exprime un lentivirus incompétent pour la réplication, dans un organe lymphoïde d'un hôte, pour la préparation d'un médicament pour amplifier une réponse immune dans l'hôte du lentivirus.

3. Utilisation selon la revendication 1 ou 2, dans laquelle le virus est défectueux au niveau de l'intégration.

4. Utilisation selon la revendication 3, dans laquelle le virus a un gène d'intégrase avec une mutation comprenant une insertion ou une délétion d'au moins trois paires de bases.

5. Utilisation selon la revendication 4, dans laquelle le gène d'intégrase est muté au site actif et au site liant l'ADN.

6. Utilisation selon la revendication 1 ou 2, dans laquelle le virus a une mutation au gène gag.

7. Utilisation selon la revendication 1 ou 2, dans laquelle le virus est choisi parmi HIV-1, HIV-2, SIV et FIV.

8. Utilisation selon l'une quelconque des revendications précédentes, dans laquelle la construction génétique est capable de transduire une cellule, ou que la cellule est transduite, pour exprimer une cytokine pour augmenter ou diriger la réponse immune de l'hôte.

9. Utilisation selon la revendication 8, dans laquelle la cytokine est choisie parmi IL-1, IL-2, IL-3, IL-4, IL-6, IL-7, IL-12, IL-15, IL-16, TNF, GM-CSF, IFN et des chimiokines de RANTES, M1P1.

10. Utilisation selon la revendication 8 ou 9, dans laquelle l'expression de la cytokine stimule une réponse immune de type Th1 ou Th2, une réponse de cellule tueuse naturelle, une réponse de cellule T cytotoxique ou une réponse de cellule B.

11. Composition pharmaceutique comprenant une cellule dendritique, qui a été transduite avec une construction génétique immunogène, qui comprend un ADN de plasmide, qui exprimer un lentivirus incompétent pour la réplication, à utiliser dans un procédé d'immunisation.

12. Composition selon la revendication 11, où le virus est tel que défini dans l'une quelconque des revendications 3-5.

13. Composition selon la revendication 11 ou 12, où le virus est tel que défini à la revendication 6 ou 7.

14. Composition selon l'une quelconque des revendications 11 à 13, où la cellule est transduite pour exprimer une cytokine telle que définie dans l'une quelconque des revendications 8 à 10.
